# EUROPEAN PATENT APPLICATION

(11) **EP 2 813 578 A1**
(43) Date of publication of application: **17.12.2014**
(21) Application number: 13172069.0
(22) Date of filing: 14.06.2013
(51) Int. Cl.: C12Q 1/68, C12Q 1/70

(54) **Methods for detecting an infectious agent, in particular HIV1, using long noncoding RNA**

(71) Applicant: Prestizia, 34820 Clapiers (FR); Centre National de la Recherche Scientifique, 75794 Paris Cedex 16 (FR); Université Montpellier 2, 34095 Montpellier Cedex 5 (FR)
(72) Inventor: Lecellier, Charles-Henri, 34920 Les Cres (FR); Blanchard, Dominique, 44300 Nantes (FR)
(74) Representative: Becker, Philippe

(57) **Abstract**

The present invention relates to compositions and methods for determining, characterizing, or monitoring a pathogenic infection in a subject. The methods and compositions of the invention are based on lncRNA analysis. The invention can be used in any mammalian, particularly in human beings.

## Description

The present invention relates to compositions and methods for detecting, monitoring, or characterizing an infection or an infectious agent in a mammalian subject. The invention also relates to novel therapies of infectious diseases. The methods and compositions of the invention are based on lncRNA analysis. The invention can be used to determine virus load or tropism, as well as anti-viral treatment efficacy, and is particularly suited for use in human beings.

### BACKGROUND OF THE INVENTION

Infectious agents are currently detected by a variety of methods, including immunologic methods (e.g., antibody-based techniques) or PCR analyses that detect specifically the infectious agent itself, or detection of specific biomarkers that reflect the presence of the infectious agent. Examples of classical biomarkers include proteins, as well as nucleic acids.

The human genome is comprised approximately 2% protein coding genes. However, more than 90% of the genome is transcribed, implying that the majority of the transcriptome is comprised of noncoding RNAs i.e., transcripts that lack an open reading frame and as such do not encode a protein. The development of novel technologies such as RNA deep sequencing has greatly improved the characterization of these ncRNAs. At this stage of the researches, ncRNAs can be classified into two main categories: small non-coding RNAs (sncRNAs) (18-200 nucleotides), which includes transcripts such as miRNAs, transfer RNAs (tRNAs), small interfering RNAs (siRNAs), piwi-interacting RNAs (piRNAs) and some ribosomal RNAs, and long non-coding RNAs (lncRNAs) (> 200 nucleotides), including pseudogenes, antisense RNA and transcribed ultraconserved regions. It is now clear that absence of protein coding capacity does not mean lack of function. In fact, this particular class of RNAs has been implicated in various biological pathways from transcription regulation to translation control and, as a consequence, ncRNA deregulations have been implicated in several pathologies (for review (Enfield et al, 2012; Guil & Esteller, 2012; Krol et al, 2010)).

At present, most of our knowledge comes from small ncRNAs. In mammals, in addition to the canonical tRNAs and snoRNAs, three novel classes of small ncRNAs have been identified (Kim et al, 2009; Rother & Meister, 2011): endogenous small interfering RNAs (endo-siRNAs), PIWI-interacting RNAs (piRNAs) and microRNAs (miRNAs). To date, a well-characterized function of endo-siRNAs and piRNAs is the maintenance of genomic integrity by silencing of transposable elements in germline cells (Kim et al, 2009; Okamura & Lai, 2008; Saito & Siomi, 2010; Senti & Brennecke, 2010). Accordingly, mammalian endo-siRNAs are highly expressed in oocytes (Watanabe et al, 2006) and embryonic stem cells (Babiarz et al, 2008; Calabrese et al, 2007) and piRNAs have consistently been reported to be essentially expressed in male and female germline cells (Saito & Siomi, 2010; Senti & Brennecke, 2010). The third class, miRNAs, recognizes mRNA targets through imperfect homologies and induces translation repression, which can be associated with mRNA deadenylation and degradation (Eulalio et al, 2009; Filipowicz et al, 2008; Flynt & Lai, 2008; Krol et al, 2010). There is over a thousand miRNAs in the human genome (1600 precursors, 2042 mature in miRBase v19) that can be predicted to modulate more than 50% of human protein-coding genes (Friedman et al, 2009) and miRNAs have been shown to play a role in almost all cellular processes.

Less information is known regarding the potential function of long non-coding RNAs. lncRNAs can be nuclear or cytoplasmic and may or may not be subject to alternative splicing or polyadenylation. Many serve vital molecular functions, including structural or trafficking roles, controlling cell cycle, differentiation, and apoptosis, and serving as precursors for smaller RNAs. Four lncRNA classes can be distinguished: Long Intergenic ncRNAs, Natural Antisense Transcripts, 3'UTR-Associated Transcripts, Enhancer RNAs and Ultraconserved Elements (Schonrock et al, 2012). However, the respective functions of lncRNAs remain rather vague.

In this regards, a number of reports indicate that lncRNAs may be regulated during the onset or development of cancers. In particular, WO2011/150453 relates to the use of the long non-coding RNA ZFASl in the diagnosis, prognosis and therapy of cancer. WO2012/018881 relates to the regulation of lncRNAs. There is however, no description in this application of any therapeutic condition in which such regulation may be useful or appropriate. US2012289581 relates to the use of lncRNAs for detecting or treating cancer, stem cell therapy, or in regenerative medicine. lncRNAs affected by HIV1 in established cell lines have been reported in Zhang et al. (mBio 4, 2013, 1). However, such lncRNAs have not been found in vivo, in biological samples.

The population of lncRNA nucleic acids has not been correlated to other pathology or physiological condition, and most attention is presently directed to miRNAs.

The present inventions stems from the unexpected discovery by the inventors that lncRNAs are modulated by infectious agents, and that lncRNAs can be used to detect, diagnose, characterize or monitor an infection in a subject.

### SUMMARY OF THE INVENTION

The inventors have surprisingly discovered that infectious pathogens such as viruses specifically modulate lncRNAs. These modulations constitute the core of novel diagnostic/prognostic tools as well as novel therapeutic targets for infectious diseases, particularly viral diseases.

The present invention therefore relates to compositions and methods for detecting, monitoring, or characterizing an infection or an infectious agent in a mammalian subject by measuring one or more lncRNAs in a sample from the subject, preferably one or more intergenic lncRNAs. The invention also relates to therapeutic methods for treating an infectious disease by modulating lncRNAs, preferably intergenic lncRNAs. The invention also relates to particular products, such as nucleic acids, primers, probes, kits and the like, that can be used to monitor lncRNAs. The invention is particularly suited to determine virus load or tropism in human subjects, as well as to develop novel anti-viral treatments.

An object of the invention relates to a method for detecting the presence or status of an infectious pathogen in a subject, comprising a determination of the presence or level of at least one lncRNA, preferably at least one intergenic lncRNA, in a biological sample from the subject, and correlating said determination to the presence or status of the infectious pathogen.

Another object of the invention relates to a method for detecting, characterizing or monitoring an infection in a subject, comprising determining the presence or level of at least one lncRNA, preferably at least one intergenic lncRNA, in a biological sample from the subject, and correlating said determination to the infection.

Another object of the invention relates to a method for detecting, characterizing or monitoring an infectious agent in a subject, comprising determining the presence or level of at least one lncRNA, preferably at least one intergenic lncRNA, in a biological sample from the subject, and correlating said determination to the presence, type or status of the infectious agent.

In a particular embodiment, the method comprises the determination of several (at least 2) distinct lncRNAs, preferably at least 2 distinct intergenic lncRNAs.

The infectious pathogen (or infectious agent) may be a virus, a bacterium, or a parasite. In this regard, the invention may be used to determine the presence or absence of the infectious pathogen, the type or serotype of an infectious pathogen, the viral load, the viral tropism, or treatment-responsiveness of a pathogen. In a particular embodiment, the pathogen is a virus, such as HIV, in particular HIV-1.

Specific examples of lncRNAs for use in the instant invention include LINC00537; LOC541471; LINC00612; LIN00422; or LINC00152.

lncRNAs may be detected using techniques known per se in the art such as by hybridization, amplification, ligand-binding, or a functional assay. Also, the lncRNA may be detected in various samples, such as cells, fluids, tissue biopsies, and the like.

A further aspect of the invention resides in a microarray comprising a nucleic acid probe specific for at least one lncRNA characteristic of a viral infection or tropism. Preferably, the microarray comprises less than 100 probes. More preferably, the microarray comprises a probe specific for at least one of the following lncRNAs: LINC00537; LOC541471; LINC00612; LIN00422; or LINC00152.

A further object of the invention resides in a set of nucleic acid primers comprising a plurality of nucleic acid primers, said plurality comprising primers that specifically amplify each lncRNAs of a lncRNA profile characteristic of a viral infection or tropism. Preferably, the set of primers comprises at least a nucleic acid primer that amplifies one (or 2, or 3, or 4, or each of) the following lncRNAs: LINC00537; LOC541471; LINC00612; LIN00422; or LINC00152.

Another object of the invention is to provide an agent that modulates a lncRNA as defined above, for use in the treatment of an infectious disease.

The present invention also relates to a method for treating a subject having an infectious disease, the method comprising determining the type, serotype, tropism or responsiveness of the infectious pathogen in said subject by a method as disclosed above, and treating the subject with a therapy adapted to the pathogen.

### BRIEF DESCRIPTION OF THE DRAWINGS

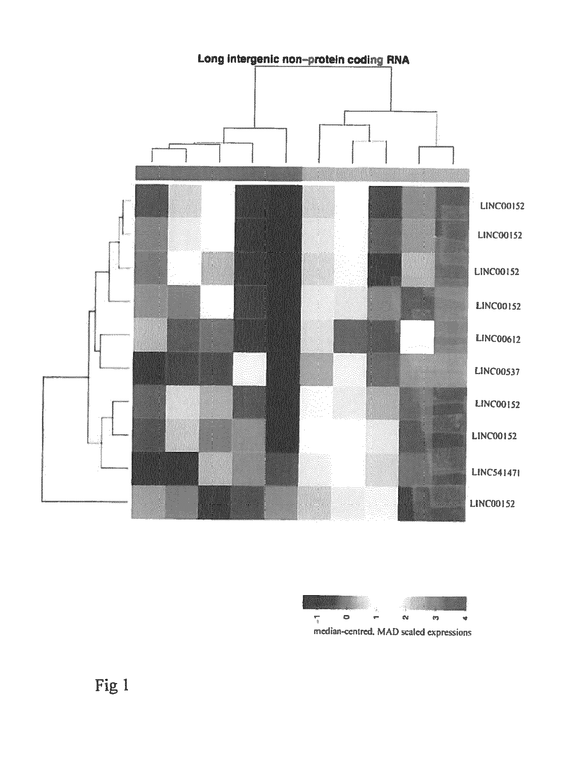

Figure 1: Heatmap analysis of lncRNAs. Several lncRNAs are specifically modulated by HIV-1.

### DETAILED DESCRIPTION OF THE INVENTION

The present invention relates to compositions and methods for detecting, monitoring, or characterizing an infection or an infectious agent in a mammalian subject based on lncRNA analysis. The invention also relates to novel therapies of infectious diseases based on lncRNA modulation. The present invention stems from the discovery that lncRNAs are modulated by infectious pathogens and can be used as biomarkers or therapeutic agents/targets for such condition. The invention can be used to determine virus load or tropism, as well as anti-viral treatment efficacy, and is particularly suited for use in human beings.

### Definitions

Within the context of this invention, the term "lncRNA" designates non-coding RNAs having a length above 200 nucleotides, typically between 200 and 20kb. lncRNAs may be found in the nucleus or cytoplasm of cells. They may be complexed with other cell components, or free. They are usually single-stranded, although they may contain 3D structures (loops, hairpins, etc.). A preferred population of lncRNAs within the context of the present invention is represented by long intergenic non coding RNAs. Indeed, the invention has shown in human biological samples that intergenic lncRNAs represent valuable biomarkers of an infection, particularly a viral infection.

The term "detecting" in relation to a pathogen or disease designates e.g., the determination of the presence, absence, location, and/or amount of the pathogen in a sample.

The term "monitoring" in relation to a pathogen or disease designates e.g., the determination of a change or evolution in the amount or location of a pathogen in a sample with time.

The term "characterizing" in relation to a pathogen or disease designates e.g., the determination of the type, serotype, or tropism of a pathogen in a sample.

Within the context of the invention, the "tropism" of a pathogen designates (i) co-receptor usage by a pathogen and/or (ii) the cell type(s) that is (are) infected by a pathogen. It is known that a number of viruses use co-receptors for cell entry. For instance, the HIV interacts with target cells through the CD4 protein and also through a second co-receptor. The main co-receptors are CXCR4 and CCR5. The co-receptor usage by the VIH reflects the progression of the immunodeficiency. VIH viruses that use CXCR4 are usually associated with the AIDS disease. Examples of further co-receptors used by VIH are CCR1, CCR2, CCR3, CCR4, CCR8, CCR9, CXCR2, or STRL33. Further information regarding these co-receptors is contained in WO2011/027075. Determining the viral tropism of a virus therefore includes, in a specific embodiment, determining co-receptor usage of a virus. In relation to the VIH, this includes more preferably determining the presence of a virus that uses CXCR4. The invention also allows the discrimination, between infectious pathogens of a same family (e.g. serotypes).

A preferred embodiment of the invention resides in a method for determining co-receptor usage by a virus. A specific and most preferred embodiment of the invention is a method for identifying HIV viruses that use CXCR4.

### Methods for determining lncRNAs

Detection of a lncRNA according to the invention includes detecting the presence (or absence) of such lncRNA or, preferably, measuring the (relative or absolute) amount of a lncRNA. Methods for detecting or measuring the amount of lncRNAs are known per se in the art. Such methods include, without limitation, quantitative reverse transcriptase polymerase chain reaction (RT-PCR), hybridization with specific probes, northern blot, affinity binding, or the like.

In a particular embodiment, lncRNAs are detected or measured in the sample by hybridization, amplification, ligand-binding, or a functional assay. In a particular embodiment, an aliquot of the biological sample is contacted with a nucleic acid probe, a nucleic acid primer, or a ligand, characteristic of at least one target lncRNAs, and the presence or amount of complexes formed between said probe, primer, or ligand and nucleic acids in the aliquot is determined. In such methods, the probe or ligand may be in solution or immobilized on a support, such as a microarray. Also, the biological sample may be treated prior to determination, e.g., diluted or concentrated or enriched for lncRNAs. The lncRNAs in the sample may also be labeled to facilitate determination.

In a particular embodiment, the method comprises (i) optionally labeling lncRNAs present in a test biological sample, and (ii) hybridizing said (optionally labeled) lncRNAs to a support (such as a microarray) comprising at least one nucleic acid probe specific for a lncRNAs characteristic of an infectious pathogen to obtain a hybridization profile, the hybridization profile being indicative of the presence, absence, type, serotype, or tropism of the infectious pathogen in the infected subject.

In another particular embodiment, the method comprises (i) obtaining lncRNAs from a test biological sample, and (ii) contacting said lncRNAs with a set of at least two, preferably at least three nucleic acid primers, said at least two, preferably at least three nucleic acid primers specifically amplifying a lncRNAs characteristic of the infectious pathogen, to obtain an amplification product, the amplification product being indicative of the presence, absence, type, serotype, or tropism of the infectious pathogen in the infected subj ect.

In a preferred embodiment, lncRNAs are determined by microarray analysis, i.e., by contacting a test sample with a microarray comprising a plurality of specific probes immobilized on its surface, allowing hybridization reaction to occur, and analyzing the hybridization profile.

To analyze lncRNAs, the first step is to obtain and/or prepare the biological sample. The test sample may be obtained from any biological sample that contains lncRNAs. In a particular embodiment, the test sample is or is obtained from a tissue sample, a biopsy, a body secretion, or a biological fluid. Because lncRNAs are essentially intracellular, the biological sample shall preferably contain cells from which lncRNAs can be analyzed. Examples of specific biological samples include, without limitation, blood, plasma, saliva, feces, urine, bronchial lavage fluid or lavage fluid from sinus, hair, etc. In a particular embodiment, the biological sample contains immune cells, particularly mononuclear cells, typically PBMCs. The invention shows lncRNAs may be characterized from circulating cells, particularly blood cells such as monocytes, macrophages, dendritic cells, lymphocytes, PBMCs. The detection of specific modulations of long non-coding RNAs in blood samples (serum, PBMC or plasma) potentially infected with a virus pave the way for the development of innovative methods aimed at characterizing contaminated samples and preventing transfusion-mediated virus transmission. Advantageously, these modulations could be directly detected in blood-bag-derived PBMCs before transfusion.

In a particular aspect, the method of the invention therefore comprises the detection of primo-infection in subjects, particularly primo HIV infection. The invention indeed allows detection of viral infection at early stage and may avoid virus contamination/transmission.

In another particular embodiment, the invention relates to a method for detecting viral infection in a blood bag, preferably in blood bag-derived PBMCs, comprising detecting the presence, amount or absence of lncRNAs as defined above in said bag or PMBCs. Such a method is typically applied before transfusion, thereby preventing transfusion-mediated virus transmission.

In this regard, another embodiment of the invention relates to a method for preventing transfusion-mediated virus transmission, comprising detecting viral infection by determining the presence or amount or absence of lncRNAs as defined above in transfusion blood bags, more preferably in transfusion blood bag-derived PBMCs, wherein blood bags positive for infection are discarded, thereby avoiding transfusion-mediated virus transmission.

Various methods exist for obtaining and preparing biological samples. In addition, collection tubes are commercially available from many sources.

Preferably, the test sample is collected and processed within 1-24 hours to minimize degradation of lncRNAs. The test sample may be frozen and processed later.

Preferably, prior to determining lncRNAs levels, the test sample is treated. Treatment may be performed e.g., to normalize lncRNAs, dilute or concentrate the sample, enrich for lncRNAs, label lncRNAs, protect RNA (e.g., inhibit RNase), etc. In a typical embodiment, and the sample is treated to release intracellular lncRNAs from cells (such as e.g., PBMCs).

Furthermore, our results also show that the sample may be frozen and subsequently thawed, without altering the reliability of the dosage. Accordingly, in a particular embodiment, the method comprises:
a) Providing a biological sample collected from a subject, the sample comprising lncRNAs,
b) Optionally treating the sample by dilution, concentration, enrichment in lncRNAs, or protection of RNAs,
c) Optionally freezing the sample, and
d) Determining lncRNAs in the sample, preferably less than 48 hours after collection or thawing of the sample, more preferably less than 24, 18, 12 or 6 hours, typically between 1-6 hours.

Steps b) and c) may be inverted. In such a case, the sample is thawed prior to the treatment step.

lncRNAs may be extracted or partially purified from the biological sample prior to determination. To that purpose, total RNAs may be purified by homogenization in the presence of a nucleic acid extraction buffer, followed by centrifugation. RNA molecules may be separated by electrophoresis on agarose gel(s) following standard techniques.

For determining a selected lncRNAs by hybridization, one or several suitable probes specific for said given lncRNAs can be produced using the nucleotide sequence of said lncRNAs. The nucleic acid sequences of lncRNAs are available from the "lncRNAs database". Preferred probes are single stranded nucleic acid molecules of 10-500 bases in length, more preferably 10-200. Most preferred probes have a length similar to that of the target lncRNAs. They contain a sequence that is complementary to the target lncRNAs, preferably a sequence that is perfectly complementary. In certain embodiments, a level of mismatch may be tolerated as long as the probe may specifically hybridize to the target lncRNAs in a complex sample, when placed under stringent conditions.

Methods for labeling DNA or RNA probes, and the conditions for hybridization thereof to target nucleic acids are known per se in the art, as described e.g., in Molecular Cloning: A Laboratory Manual, J. Sambrook et al., eds., 2nd edition, Cold Spring Harbor Laboratory Press, 1989, incorporated therein by reference.

The relative number of lncRNAs in a sample can also be determined by specific amplification. Various techniques exist for amplifying lncRNAs nucleic acid sequences, including without limitation reverse transcription (RT), polymerase chain reaction (PCR), real-time PCR (quantitative PCR (q-PCR)), nucleic acid sequence-base amplification, multiplex ligatable probe amplification, rolling circle amplification, or strand displacement amplification. In a preferred embodiment, the determination is made by reverse transcription of lncRNAs, followed by amplification of the reverse-transcribed transcripts by polymerase chain reaction (RT-PCR). Amplification generally uses nucleic acid primers. A primer is generally about 15 to 40 nucleotides in length, single stranded, and contains a region that is complementary to a portion of the target lncRNAs. Generally, a pair of primers is used comprising: a forward primer that can anneal to the target lncRNAs, and a reverse primer that is designed to anneal to the complement of the reverse transcribed target lncRNAs.

The invention may involve the detection of the presence or absence of one or several lncRNAs in a sample, and a correlation of said presence or absence to the presence, absence or characteristic of a pathogen. The invention may also or alternatively involve a determination of the amount (or level) of one or several lncRNAs, and a correlation of said amount to the presence, absence or characteristic of a pathogen. Correlating the amount to the pathogen may comprise, for instance, a comparison of said amount to a control or reference value. The control or reference may be an external control, such as a lncRNAs in a test sample from a subject known to be non-infected by the pathogenic agent, or known to be infected by the pathogenic agent, or to both. The external control may be e.g., a known amount of a synthetic RNA. The control may be a pooled, average, or individual sample. The control or reference value may be a mean or average reference value for a given lncRNAs in a reference situation.

In some embodiments, it is desirable to simultaneously determine the expression level of a plurality of different lncRNAs in the test sample. In certain instances, it may even be desirable to determine the expression level of all known lncRNAs. Assessing expression levels for several lncRNAs may be performed using microarray or biochips comprising a plurality of probes or using a combination of various primers. The use of microarray or primers has many advantages for lncRNAs detection. Indeed, several hundreds of lncRNAs can be identified in a single sample at one time point. Moreover, a small amount of RNA is needed. Assessing expression levels for several lncRNAs may also be performed using specific stem-loop RT followed by quantitative PCRs, particularly low density RT-qPCR like TLDA (TaqMan® Low Density Arrays; Life Technologies). As disclosed in the experimental section, such method can be used efficiently to assess simultaneously a large number of lncRNAs in a complex test sample. The method may therefore comprise the determination of at least 2, 3, 4, 5, 10, 15, 20, 30, 40, 50 or even more lncRNAs.

In this regard, the invention also relates to lncRNAs or lncRNAs profiles characteristic of an infectious disease.

### lncRNAs correlated to viral infection in human samples

The inventors have identified specific intergenic lncRNAs correlated to the presence or characteristic (e.g., tropism) of a virus, particularly VIH, in biological samples obtained from human subjects. These include, preferably, LINC00537; LOC541471; LINC00612; LIN00422; or LINC00152. As disclosed in the examples, these lncRNAs are modulated in subjects infected by VIH and may be used to (i) detect the presence/absence of the virus and/or (ii) to characterize the virus tropism and serotype.

The nucleic acid sequence of these lncRNAs is available from the lncRNA databasis, as indicated above. As an illustration, their nucleic acid sequence is provided below:
LINC00537, SEQ ID NO: 1 
GAAACAGACAAATATCAAAAAAGACAAAGAAGGGCATTACATAATGGTAAAGGTTTCAGTTCAA
CAAAGAAAAATCTCTAACTATTATAAATATATAAGCACCCAACACAGGAGCACCC
LOC541471, ENST00000409054, SEQ ID NO: 2 
ACAAGTATGAAGAGAATGTCGGGAGAGGAAGTGGTGGCTATGAGTCAGCATGAGTCATCT
CGTTCCAATGAGAATGAAGGCTGAGGTGTGCGCCTTTTTTTTTTTTCCTTCTTAGTCGT
GTGTACATCATTGGGAATGGAGGGAAATAAATGACTGGATGGTCGCTGCTTTTTAAGTTT
CAAATTGACATTCCAGACAAGCGGTGCCTGAGCCCGTGCCTGTCTTCAGATCTTCACAGC
ACAGTTCCTGGGAAGGTGGAGCCACCAGCCTCTCCTTGACAAGCAAAGTGGATCAGCAAA
GGCTGCAGTCACCAGCATCTTTTCCAACCTTAATGAACTGTATCCTCAAAAGAACACTAT
CAGACTGGTAAGACAAGCATTATTGTCCCACTTCACAGATGAAGAGCTTGGGGGCCCTGT
GGATGTGTAGGAGAGTCGGCCTTCCTTTTTATAGGCCTTTTGCTGAGCTCAGAGGACAGA
GCAGAAGACAAAGCCGAAAGCAGCCCTAAGCAGACACTATCCCAGCCCAAGGAGCTATGC
AGACAGCTCCTGCCTAGAGGAGAAACAATTCTCTCCTTCAGCCAGGCATGTGGCACAGAG
GTGAACAGGAAATGCTTAGGGAGACAGTTCACAGATATATATGTACTTCACAGGGCACAA
TTTAATCCATAAATCAATCATGGATCACTGCTAAAGAAAACAAAATATGCTGCCACTAAT
TTGCCACCACCCTGTGAGCATGATTGGATGATGTTTATTTGATTAGGAGATTGCTCCAT
TTATAAATCTTCAATACATCCTGTCCCCCTAAAACGGCATCTGGATCTTTTGAGGGTTAA
AAAAAAAAAAAGTGAAATTGGATGAGGGATGAGAGCTGGACTTCTGTGTGTCCCGGGGTC
TCTGGATCCACAGCCTGGTGCCTAGCATCAGTTAAGCCCACGCTGGGGTCGCATCACATC
CCGCAAAGCCACGTGCTCTGTGAGGCCAACAGGAGCCCCGCCGGGTGGACCAGCTGGAAT
CTCAGAGAGCGCCGACTCTGAAAATACCCGGCTCTGCAGAAGCACGCTGGGCCCAGGGGG
CTTCTAGACTGACAGC
LINC00612, ENST00000538094, SEQ ID NO: 3 
GCCCCGGGGCTCAGGGGCAGCCCAGGACTCTCCCTCGCCCGGCTCACGGCCCTGCCTGGG
GGGCGCCTCCGGGAGCAGATCACGAGCCCTGGGGCTCAGCCCTCAGGCGCGTCTAGCTCG
GCGGTCACCCCAGTGCCGGGAGGACCCTGAAGACGCGCCCCAGGCGGCCCTACTCAGAGC
 
CTCCTCCCAAGGCCCAGGAATGCGGCGCTGTCGGAGGTGTGCGCGGTGGCCACACCGCTG
TCCGGGTCCCCAAAGCGGGCCCCGCTCCCACTTCTCGCCTTGGCCCCGAACCCTGGGTCC
AGCCCCAGCGCTTTGTGTGCGAACACCGCTCCGCCCCGGACCCAGCTCCGCCTTGGGGCC
CCTCTCTGCCTGCCCCTCCGTGCCCGACTACACTGCTTCCCCTCCGGCGGGCGACTCAGC
CCGGTCCATCGTGGCGGCTTCCAGGGCGGCAGGCTCCGGGAGTACTCCCGGGGCCGGCTC
CAAGGACTGTTCCCCTCCTCCCCACTCCCACTCCGCGGCGGCGGCGGGCGAGAGCGGCGA
CATAGGGCCAGGGTCCGGAGCGGTGGAGGCTCCTGGCCGGGGAGCACGTCGCCCCACCCG
GCAACGCGAGGATGGTGGCGGCGCAGTCGGCTGCTTTGGGGTCTCAAGGCACAGGGGACG
CGAGGCACAGATGTCCCACAGCAGCCACTGCGGCTCCCGCAGCTGCTCCGCCGCCGCTGC
CCGCCCCTCCCTGCTGCAGCTGGCGTGATGGCAGCGGCAGCTCTGGACGCCCCACTGCTG
CCACCATCAGCCTTGTGAAATAGCTTGAAAGATCTAGCCACGTGAGCAGGACAGAAGTGC
ACAACAACCATATCTTGATTTCTGTGGACCAGGTGGGGCCCCTGCCAGCCTTGCTAGACA
GACCCAGGTGAACAGTCCTTCTAGGGGATCTCATCACCAGGCAAGCACGTGGTACGAGAA
GAGCAGTCATTAGGAAGGCCATTTGGAAAAGCACATCCTCTCTGTTCACGTGAGATATTT
TACATCCTCATTCCTCATCGCAAGCTTCCTGGGATTTGGAGTGTCACAGACAAGAGGGTT
GGGGGAGGCCAGTAGGTATGGATTTGTTTATATTAAAATGAGCATATGAATATTTATATG
TTTATATTAAAACATATATGTTTGTTTATATTACAATGAGCATATGAATATTTATATGTT
TATATTAAAACATATATGTTTGTTTATATTAAAATGAGCATATGAATATTTCTGTATACT
TCAGATAAACATTCTTTTCCATAAATAAGCTTCATCATCCAGAAGCCATGTTGAAAGTTG
GTAATCAAGGATAGGAAGTGTTTCCAAGGGTTGTCAGTGATTAAATCAACCTTACCTTAG
CATACATGTATGAGAGAAGTTAGATAAATTATACATAGAGAGATAGCTAGATAGATAATA
GATAAATAAGGTAGATGACAGATTAGATACAGATGATAAACAGATATGCTATCAGTCTAG
ATCTATTACTCACAGTCAGTCGTATCACAGTGTAATTCCATCAACTTCATTTGCAGCAAA
AGAACAGCCCCTGATGTACGCCTGTTTCCTGAATATTTGGAAATATTAATATTAATATAG
ATAGAGTCAGCTTGCCAGTATAAAGAATCTATCTCTCACTATAGGCAGAGCCATGTGTTG
GATATTATGGAGGAATATGTATTATTTTAATCGTTTAAAAGACTAATAAACTTCTAATCT
ACTTTTGGGAGCCTCTGCTCAAGTTCCTGAGGTTCCTACTCTAAAATCCACATCACCCTC
CAACTGCTCCCTATGTGAGCCATTAGGGAGCACAATGAATTAGAGAATGAATCCTATTAG
CCCACTAAAAAATTATCTTTCAAAGGATGAAGACTGATGCTAAGGAAAAAGAGATGAGGC
CTCCCTGGTTCCGCATTATTACTCCTTCCTTTCTTGGGTTACGGATTGAACACTGACCTT
TTCTTTCACTCTTCTGCAAGACAACCCCATAAGAAAGCTGAAACCTGCTTTTGTGAAAGC
CTATGATTACTATAAGACAGGCACAGAGCAAGAAAATGTTTGAGGCTTGTGTCTATAAAC
AAGACTTGGCTGGAATCCCCAGACCCACGACTTTTAAAGGATATTTGTCCCTGCAAT
ACAGATACTGAATGAGGTTGGCAAATAAATATGTATTTCTAGAGACTCTATAA
LINC00422, ENST00000434601, SEQ ID NO: 4 
TCATCCTGTCCTCACAATGGCTGGTGTGGAGACCCACAGACTTCATCCTTAGGACGGACC
TTCAGGCACCCAGGCTGCCTACTGACCTCCCTGGCATGGTGGAACCGGAGCTCGTTG
GTAAGCAGGAGGATTCAGGACTATCGACTGAACCCCACCTCCTCTGTGGCACGCACCGTG
CTGTGTCTTTGCAGTCATCATCTCATCACCTCTGCACAGGAAGATTATCTCCCCCATTTT
GTGATGAGGAAATGGAGGCTCCAGGTGGAAAGATGCAACCTGGGCCAGAGGCCCCTGGAG
GTTGTGCCTGAACCCAGACAGATCACACAAACCTGAGCAGCCATTAGCCGCTGTGTGGGC
TCCTTTGCACTGTGGGGCTGCTCCTGGGGATATTTACGTTGTGAACTGGTAAAATCTGAA
CTGCAGAATATCAATTTCCAGACATTCTTCCTAGATGATGCCGTCTGCCACGGAGACCAT
GTAGATGGATTGAATGGGGATATGATGAGGATGACCACCCCTGCATTTGCTAAGTTCTTG
GGAGTGGCATCGATCTCAGCCATTCCACTGGAATAGGGTCAGGAAACAAAACGTGAGGGC
TCTACCAGCTGCTCAGTCTATCCTTTCCAATTGAGCACACTGGAACTGAGGAAGCAAAGA
CAGGGTGAGCCCTAACACCCTCATCATCACTGCTGAGCCTGCCTTGACAAGAACTCCATT
TATCACCCGCCTCCATAATAGCCCATTCTAACTAGCTGTCATCGTGGCATTTCAGGTTCT
GCTGATGTCATTATAATGGACCTTAACTAGTGGGCTGAAGGCAATGAGGGGATGTGAGGA
TGCATCTTGTGAAGGACAAAAGTCATCCTGTAAGGATTCATGAGACCTGAGCAGCCAGCC
AGCCCTGAAATGGTTGGAACAGGAGAAAGAAGAGCTCGACAGATACCCTGCCTGGGC
 
TGGCAGCTAGCTGCACCACAAGCTGGCCAGCTGACAGCGGGCACATCCCTTCATCACCCA
GTGCCTTTCTTCCCTCTTGTGAAAATGGAGATATGCTGAGAAGGTTACTTCAGCTCATCC
AGAGAAAGCTTCTTCAGCTAACTAGGCTGTAGAAAGGGCCTACAGCCTAAATTTTCAAAA
ACAAGTTAATGCCAGAATATAAACTCCGTGAAGTAACA
LINC00152, ENST00000409054, SEQ ID NO: 5

ACAAGTATGAAGAGAATGTCGGGAGAGGAAGTGGTGGCTATGAGTCAGCATGAGTCATCT
CGTTCCAATGAGAATGAAGGCTGAGGTGTGCGCCTTTTTTTTTTTTCCTTCTTAGTCGT
GTGTACATCATTGGGAATGGAGGGAAATAAATGACTGGATGGTCGCTGCTTTTTAAGTTT
CAAATTGACATTCCAGACAAGCGGTGCCTGAGCCCGTGCCTGTCTTCAGATCTTCACAGC
ACAGTTCCTGGGAAGGTGGAGCCACCAGCCTCTCCTTGACAAGCAAAGTGGATCAGCAAA
GGCTGCAGTCACCAGCATCTTTTCCAACCTTAATGAACTGTATCCTCAAAAGAACACTAT
CAGACTGGTAAGACAAGCATTATTGTCCCACTTCACAGATGAAGAGCTTGGGGGCCCTGT
GGATGTGTAGGAGAGTCGGCCTTCCTTTTTATAGGCCTTTTGCTGAGCTCAGAGGACAGA
GCAGAAGACAAAGCCGAAAGCAGCCCTAAGCAGACACTATCCCAGCCCAAGGAGCTATGC
AGACAGCTCCTGCCTAGAGGAGAAACAATTCTCTCCTTCAGCCAGGCATGTGGCACAGAG
GTGAACAGGAAATGCTTAGGGAGACAGTTCACAGATATATATGTACTTCACAGGGCACAA
TTTAATCCATAAATCAATCATGGATCACTGCTAAAGAAAACAAAATATGCTGCCACTAAT
TTGCCACCACCCTGTGAGCATGATTGGATGATGTTTATTTGATTAGGAGATTGCTCCAT
TTATAAATCTTCAATACATCCTGTCCCCCTAAAACGGCATCTGGATCTTTTGAGGGTTAA
AAAAAAAAAAAGTGAAATTGGATGAGGGATGAGAGCTGGACTTCTGTGTGTCCCGGGGTC
TCTGGATCCACAGCCTGGTGCCTAGCATCAGTTAAGCCCACGCTGGGGTCGCATCACATC
CCGCAAAGCCACGTGCTCTGTGAGGCCAACAGGAGCCCCGCCGGGTGGACCAGCTGGAAT
CTCAGAGAGCGCCGACTCTGAAAATACCCGGCTCTGCAGAAGCACGCTGGGCCCAGGGGG
CTTCTAGACTGACAGC

Detection/quantification of these lncRNAs in a sample can be made following the techniques disclosed above, using specific nucleic acid probes or primers comprising all or part (e.g., between 10-200) nucleotides of the above sequences or of the sequence complementary thereto.

It should be noted that further lncRNAs characteristic of a virus can be identified following the teaching of the present invention. In this regard, the invention relates to a method of identifying or generating a lncRNA or a lncRNA profile characteristic of an infectious pathogen or disease, comprising:
(i) obtaining or generating a population of lncRNAs from a biological sample of a subject infected with a pathogen, and
(ii) comparing said population to a reference lncRNA population obtained or generated from a sample of a subject not infected with said pathogen, or infected with a pathogen having a different characteristic,
   wherein the lncRNAs distinctive between said two populations represent a lncRNA or lncRNA profile characteristic of the infectious pathogen or disease.

In a preferred embodiment, the invention relates to a method for detecting a lncRNA selected from LINC00537; LOC541471; LINC00612; LIN00422; or LINC00152 in a sample, the method comprising obtaining a test sample, optionally treating the sample to disrupt or lyse cells, and assessing the presence or amount of at least one a lncRNA selected from LINC00537 ; LOC541471 ; LINC00612; LIN00422; or LINC00152. In a particular embodiment, the method comprises assessing the presence or amount of at least two of the above lncRNAs.

A specific object of the invention also relates to a method for detecting the presence or absence of HIV in a subject, comprising determining in a test sample from the subject the presence or amount of at least one lncRNA selected from LINC00537; LOC541471; LINC00612; LIN00422; or LINC00152, said determination being correlated to the presence or absence of HIV.

A further specific object of the invention relates to a method for detecting the presence or absence of HIV1 in a subject, comprising determining in a test sample from the subject the presence or amount of at least one lncRNA selected from LINC00537; LOC541471; LINC00612; LIN00422; or LINC00152, said determination being correlated to the presence or absence of HIV1.

Another specific object of the invention also relates to a method for detecting the presence or absence of HIV in a subject that uses the CXCR4 or the CCR5 co-receptor, comprising determining in a test sample from the subject the presence or amount of at least one lncRNA selected from LINC00537; LOC541471; LINC00612; LIN00422; or LINC00152, said determination being correlated to CXCR4 or CCR5 receptor usage by VIH.

Preferably, the method comprises determining a lncRNA selected from LOC541471, LINC00152, or LINC00422, wherein LOC541471 or LINC00152 is indicative of CCR5 co-receptor usage by the virus, and LINC00422 is indicative of CXCR4 co-receptor usage by the virus.

In a particular aspect of the method of the invention, the lncRNA is detected in a sample of blood comprising e.g., circulating PBMCs, granulocytes, platelets and/or red cells. In a particular embodiment, the invention therefore comprises a step of isolating such cells from the test sample and assessing the amount or presence of lncRNA in said cells, typically after cell disruption.

In a particular aspect, the invention relates to a method for detecting viral infection in a blood bag, preferably in blood bag-derived PBMCs, comprising detecting the presence, amount or absence of lncRNAs as defined above in said bag or PMBCs. Such a method is typically performed before transfusion, thereby preventing transfusion-mediated virus transmission.

In this regard, another embodiment of the invention relates to a method for preventing transfusion-mediated virus transmission, comprising detecting viral infection by determining the presence or amount or absence of lncRNAs as defined above in transfusion blood bags, more preferably in transfusion blood bag-derived PBMCs, wherein blood bags positive for infection are discarded, thereby avoiding transfusion-mediated virus transmission.

The invention further relates to a microarray comprising a nucleic acid probe specific for at least one lncRNA characteristic of a pathogen, preferably a nucleic acid probe specific for at least one lncRNA selected from LOC541471, LINC00152, or LINC00422. In a particular embodiment, the microarray comprises a plurality of nucleic acid probes, said plurality of probes comprising probes specific for each lncRNA of a lncRNA profile characteristic of a pathogen. In a further particular embodiment, the invention relates to a microarray comprising a probe specific for each of the following lncRNAs: LOC541471, LINC00152, and LINC00422. In the microarray, the probes are preferably immobilized on a surface, typically in an ordered arrangement.

The invention also relates to a composition comprising a plurality of nucleic acid primers, said plurality comprising at least one primer that specifically amplify at least one lncRNA characteristic of a pathogen, preferably selected from LOC541471, LINC00152, or LINC00422. In a particular embodiment, the composition comprises a plurality of primers that specifically amplify at least one, preferably at least two, even more preferably at least 3, 4, or 5 lncRNAs characteristic of a pathogen, preferably selected from LOC541471, LINC00152, or LINC00422.

The invention also relates to a composition or set of nucleic acid primers comprising a plurality of nucleic acid primers, said plurality comprising primers that specifically amplify each lncRNA of a lncRNA profile characteristic of a pathogen.

The present invention further relates to a kit containing a microarray as defined above.

The kit may further contain reagents for a hybridization or amplification reaction, and/or a control sample, and/or a manual of instructions, and the like.

The present invention can be used to determine the presence or characteristics of any virus. It is particularly suited to determine the presence or tropism of viruses that infect eukaryotic cells, particularly mammalian cells (e.g., human cells, canine cells, cat cells, avian cells, or murine cells, for instance). The invention is particularly adapted to determine the presence or tropism of viruses that infect human beings.

Examples of viruses include, without limitation, retroviruses, herpes viruses, adenoviruses, enteroviruses, reoviruses, papillomaviruses, picornaviruses, pox viruses, flaviviruses, etc. Specific examples for which a specific tropism may be identified according to the invention include the following viruses: Human Immunodeficiency Virus (HIV-1 and HIV-2), the hepatitis A, B or C virus, Delta hepatitis virus, occult B hepatitis virus, measle virus, herpes virus (including HSV-1, HSV2, HSV-6, EBV and CMV), papillomaviruses, rotavirus, parvovirus, influenza virus, parainfluenza virus, rhinovirus, coronavirus, , poxvirus, rotavirus, HTLV-1, HTLV-2, dengue virus, West Nile virus, Yellow fever virus, varicella zoster virus, SRAS, respiratory sincytial virus, Chikungunya virus, or hemorragic fever viruses (*Arenaviridae, Filoviridae, Bunyaviridae, Flaviviridae);* rubella virus, mumps virus, polio virus.

### lncRNAs correlated to bacterial infection

The invention further relates to a method of identifying or generating a lncRNA or a lncRNA profile characteristic of a bacterium or bacterial disease, comprising:
(i) generating or obtaining a population of lncRNAs from a biological sample of a subject infected with a bacterium, and
(ii) comparing said population to a reference lncRNA population obtained or generated from a sample of a subject not infected with said bacterium, or infected with a bacterium having a different characteristic,
wherein the lncRNAs distinctive between said two populations represent a lncRNA or lncRNA profile characteristic of the bacterium or bacterial disease.

Examples of bacterial pathogens include, e.g., E coli, staphylococcus, etc.

### Therapy of infectious diseases by lncRNA modulation

As indicated above, the invention also provides novel therapeutic approaches for treating infectious diseases based on a modulation of lncRNAs.

In this regard, the present invention also relates to a method for treating a subject having an infectious disease, the method comprising determining the characteristic (e.g., type, serotype, tropism or responsiveness) of the infectious pathogen in said subject by a method as disclosed above, and treating the subject with a therapy adapted to the pathogen.

The invention also relates to an agent that modulates (the expression and/or activity of) a lncRNA, for use in the treatment of an infectious disease.

The invention also relates to a method for treating an infectious disease, comprising administering to the subject an agent that modulates (the expression and/or activity of) a lncRNA, such modulation leading to a treatment of said disease.

The invention further relates to a method of designing, engineering, screening for, or otherwise producing therapeutic agent, said method including the step of identifying a candidate agent which at least partly modulates the expression and/or activity of a lncRNA.

The agent may be any compound (nucleic acid, chemical agent, peptide, etc) that modulates (e.g., increases, or mimics, or inhibits) the expression or activity of a lncRNA as defined above, preferably a lncRNA selected from LINC00537 ; LOC541471 ; LINC00612; LIN00422; or LINC00152.

The compound is typically a nucleic acid comprising the sequence of all or a functional part of a lncRNA as listed above, or a nucleic acid comprising a nucleotide sequence having at least 70% identity thereto; or a nucleic acid having a sequence complementary thereto.

The invention also relates to a pharmaceutical composition comprising a therapeutic agent as defined above and a pharmaceutically acceptable excipient.

Further aspects and advantages of the invention will be disclosed in the following experimental section, which should be considered as illustrative. The contents of all references, patents and published patent applications cited throughout this application are incorporated herein by reference.

### EXAMPLES

We herein describe methods for identifying or characterizing infectious pathogens, especially the HIV (Human Immunodeficiency virus) by measuring lncRNAs in biological samples.

### 1. Biological samples

### 1.1. Serum and plasma

Serum is the non-cellular portion of coagulated blood. Plasma also does not contain peripheral blood cells, but unlike serum, plasma contains clotting factors.

Serum or plasma samples are obtained from voluntary blood donors collected at the Etablissement Français du Sang of Montpellier, or from human patients previously screened for HIV contamination.

Blood collection tubes are commercially available from many sources and in a variety of formats (e.g., Becton Dickenson Vacutainer tubes-SST(TM), glass serum tubes, or plastic serum tubes).

Four to six mL of blood are withdrawn from controls or patients by venipuncture into collection tubes using standard methods. It is allowed to clot for 4 to 6 hours at room temperature and then keep at 4-8°C. When using plasma, blood is collected into EDTA blood tubes (Sarstedt, Monovette EDTA K) containing 1.6 mg EDTA. The EDTA prevents coagulation. Tubes were inverted 8-10 times immediately after blood collection

The serum or the plasma is then separated from the cellular portion of the coagulated or EDTA_treated blood, respectively, by centrifugation. Centrifugation to prepare serum or plasma is usually performed at a speed of 500 x g, 10 min.

Serum and plasma were aliquoted in 2mL microtubes and frozen at -80°C until it was subjected to RNA isolation. Alternatively, microRNAs were extracted using the appropriate kits and frozen under aliquots at -80°C.

### 1.2. Peripheral blood cells

Peripheral blood mononuclear cells are components of peripheral blood that can be easily isolated from the blood sample. They were isolated from fresh blood by Ficoll gradient separation and counted.

Then, 1 x 10E6 cells were mixed with the indicated volume of the lysis buffer of the kit, following manufacturer instructions, to achieve lysis and inactivate endogenous RNAses.

Lysates were frozen at -80° C until RNA purification. Alternatively, lncRNAs were extracted using the appropriate kits and frozen under aliquots at -80°C.

### 2. Extraction and analysis of IncRNA

lncRNAs may be extracted and purified using methods known per se in the art. Many methods are known for isolating total RNA, or for specifically extracting small RNAs, including miRNAs. The RNA may be extracted using commercially available kits (e.g., Norgen, Ambion, Life Technology, Agilent, Sigma, Qiagen, Roche, Texagen, Macherey Nagel, Amresco, Epicentre, ZymoReserach, BioMobile).

Total RNAs were extracted from human PBMCs using the Macherey-Nagel extraction kit. We collected 5 samples from non-infected individuals and 5 samples from HIV-infected patients. Clinical details are provided in the table below. Collection was performed using 1.10E6 PMBCs.

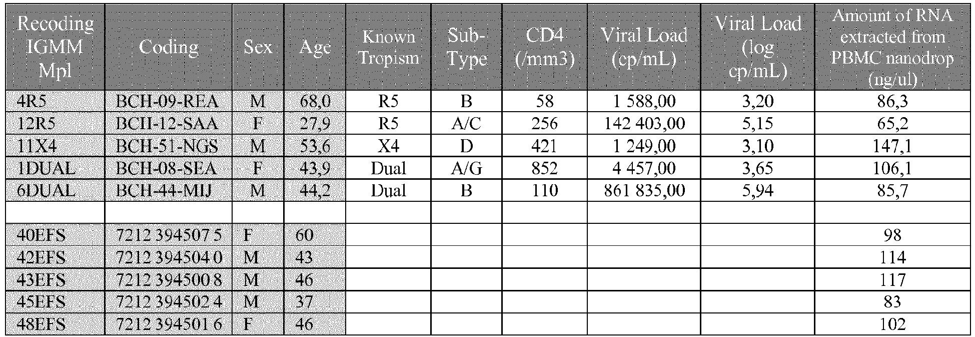

RNA sample integrity was assessed using RNA 6000 Nano Assay (based on http://www.icmb.utexas.edu/core/DNA/Information_Sheets/Bioanalyzer/RNA_6000_N ano_Assay.pdf) and Bioanalyzer 2100 (Agilent technologies). High quality RNAs with a ribosomal ratio greater than 1.9 and no evidence of degradation were used in this study. Total RNAs were analyzed using the Affymetrix GeneChip® Human Gene 2.0 ST Array (http://www.affymetrix.com/estore/browse/products.jsp?productId=131453#1_1). This chip contains probes corresponding to 11,086 lncRNAs, according to sequences and transcripts listed in the lncRNA database (Amaral et al, 2011).

Microarray images were quantified using the GenePix Pro 6.1 software (Molecular Devices, Sunnyvale, CA). GenePix flagging, able to filter out low-quality spots, was optimized to reduce the number of false negative and false positive spots in the analysis. Information was imported from GenePix Results (GPR) files, processed and further analyzed by R/Bioconductor tools. Several normalization methods were applied to remove experimental bias in the dataset. Spatial effects were removed using two-dimensional approximation of zero log-ratio level by radial basis functions. Dye-effect was removed by a Lowess normalization with the smoothing parameter f=0.67. Next, between-array normalization was performed by median-based centring and scaling of log-ratio distributions. The quality of microarrays was controlled by distributions of log-ratio values of the good-flagged spots, total number of good-flagged spots per array, average correlation with other arrays and spatial homogeneity. Replicate spots were additionally filtered and summarized before the statistical analysis: only spots with intensities significantly higher than the background level were considered. To find genes with statistically significant regulation in each class (HIV-1 vs. Non Infected cells (NI) in the first analysis; X4 vs. NI, R5 vs. NI and DUAL vs. NI in the second analysis) the empirical Bayes method (Smyth, 2004 PMID 16646809), implemented in R/Bioconductor's limma package, was used. Genes with adjusted p-value lower than 0.05 were considered as significantly regulated.

### 3. Identification of IncRNAs modulated by HIV infection

Several lncRNAs were identified which are modulated by HIV-1.

These lncRNAs are disclosed in Figure 1, which depicts a heatmap of a LIMMA's empirical Bayes statistics comparing HIV- and HIV+ samples (sample names are indicated at the bottom of the figure). Only LINC significantly modulated in HIV+ samples are indicated (adj p-val <0.05, see table 1). The fold changes are shown using the color code indicated at the bottom of the figure.

This statistical analysis shows that the expression of several lncRNAs are able to discriminate non infected PBMCs (5 first samples in blue) from HIV-1-infected PBMCs (considering X4, R5 or DUAL-tropic HIVs, five last samples in red).

Details regarding these lncRNAs are also provided in table 1. Data were preprocessed by Partek GS 6.6 using RMA with GC correction and quantile normalization. Statistical analysis used two complementary approaches: ANOVA-based analysis with step-up FDR in Partek and limma-based analysis in R/Bioconductor.

These results show that at least 4 lncRNAs are significantly modulated by HIV-1 (adjusted pval<0.05). These lncRNAs are also specifically modulated by HIV-1, i.e., they are not significantly modulated by a distinct pathogen.

These 4 lncRNAs, LINC00537, LOC541471, LINC00612, and LINC00152, as well as Inc LINC00422, represent valuable biomarkers for detecting HIV1 infection in human subjects.

### 4. Identification of IncRNAs specific for HIV-1/CCR5

lncRNAs that are specifically modulated in human subjects infected by HIV having a particular co-receptor usage have been identified. More particularly, lncRNAs specifically modulated by HIV1 with tropism for CCR5 co-receptor have been identified:
LOC541471
LINC00152

### 5. Identification of IncRNAs specific for HIV-1/X4

lncRNAs that are specifically modulated in human subjects infected by HIV having a particular co-receptor usage have been identified. More particularly, lncRNAs specifically modulated by HIV1 with tropism for X4 co-receptor have been identified:
LINC00422

### Discussion

The results show lncRNAs characteristic of pathogenic infection can be efficiently detected in human samples. Our results also show that lncRNAs may be identified which are specific for viral tropism and may be used to efficiently discriminate patients or selected most appropriate treatment regimen.

**Table 1**

| **logFC** | **AveExpr** | **Adj.P.Val** | **Gene.Symbol** | **Gene_assignment** |
|---|---|---|---|---|
| 1.1879 | 6.0073 | 2.114e-03 | LINC00537 | BC110369 // LINC00537 // long intergenic non-protein coding RNA 537 |
| 1.2832 | 5.5582 | 1.295e-02 | LOC541471 | ENST00000409054 // LOC541471 // uncharaterized LOC541471//2q1 |
| 0.5379 | 5.2651 | 1.296e-02 | LINC00612 | BC028195 // LINC00612 // long intergenic non-protein coding RNA 612 |
| 1.1118 | 8.0994 | 3.285e-02 | LINC00152 | BC070202 // LINC00152 // long intergenic non-protein coding RNA 152 |
| 0.8969 | 8.2916 | 5.346e-02 | LINC00152 | ENST00000424851 // LINC00152 // long intergenic non-protein coding RNA 152 |
| 0.6921 | 7.4065 | 5.346e-02 | LINC00152 | ENST00000453478 // LINC00152 // long intergenic non-protein coding RNA 152 |
| 1.0957 | 5.8715 | 5.578e-02 | LINC00152 | ENST00000443467 // LINC00152 // long intergenic non-protein coding RNA 152 |
| 0.9013 | 8.1976 | 5.578e-02 | LINC00152 | ENST00000419736 // LINC00152 // long intergenic non-protein coding RNA 152 |
| 0.8079 | 7.4637 | 5.578e-02 | LINC00152 | ENST00000419736 // LINC00152 // long intergenic non-protein coding RNA 152 |
| 0.7018 | 8.2190 | 5.578e-02 | LINC00152 | ENST00000419736 // LINC00152 // long intergenic non-protein coding RNA 152 |
| 0.6634 | 7.7909 | 5.578e-02 | LINC00152 | ENST00000444301 // LINC00152 // long intergenic non-protein coding RNA 152 |
| 0.5795 | 9.1798 | 5.578e-02 | LINC00152 | ENST00000409569 // LINC00152 // long intergenic non-protein coding RNA 152 |
| 0.5644 | 6.4035 | 5.578e-02 | LINC00152 | ENST00000432818 // LINC00152 // long intergenic non-protein coding RNA 152 |
| 0.7778 | 7.9685 | 8.390e-02 | LOC541471 | BX647931 // LOC541471 // uncharacterized LOC541471 // 2q13 // 5414 |

| | | | | |
|---|---|---|---|---|
| The adjusted p-value is indicated | | | | |

## Claims

1. An *in vitro* method for detecting, monitoring or characterizing an infectious pathogen in a subject, comprising a determination of the presence or level of at least one lncRNA in a biological sample from the subject, and correlating said determination to the infectious pathogen.

2. The method of claim 1, comprising determining the presence or level of several lncRNAs in the biological sample from the subject, and correlating said determination to the infectious pathogen.

3. The method of claim 1 or 2, wherein said correlating step comprises comparing said determination to at least a reference value or profile.

4. The method of claim 1 or 2, wherein the infectious pathogen is a virus, a bacterium, or a parasite.

5. The method of any one of claims 1 to 4, for detecting the presence or absence of an infectious pathogen in a subject, preferably of a primo-infection, or for determining the type or serotype of an infectious pathogen in a subject infected by a pathogen, preferably of a virus, or for determining the viral load in a subject infected by a virus, or for monitoring the viral load in the subject during treatment.

6. The method of any one of claims 1 to 4, for detecting the presence or absence of a virus in a blood bag.

7. The method of any one of claims 1 to 4, for determining the viral tropism in a subject infected by a virus.

8. The method of any one of claims 1 to 4, for determining the efficacy of a drug or candidate drug in a subject infected by a pathogen, by determining the presence or level of at least one lncRNA in a biological sample from the subject at various time points during treatment.

9. The method of any one of the preceding claims, wherein the pathogen is human immunodeficiency virus (HIV), particularly HIV-1.

10. The method of claim 9, for determining whether a subject is infected with a HIV, particularly HIV-1.

11. The method of claim 9, for determining whether the HIV in an infected subject uses the CXCR4 or the CCR5 co-receptor.

12. The method claim 10, comprising determining in a sample from the subject the level of at least one lncRNA selected from LINC00537; LOC541471; LINC00612; LIN00422; or LINC00152.

13. The method of claim 11, comprising determining the level of a lncRNA selected from LOC541471, LINC00152, or LINC00422, wherein LOC541471 or LINC00152 is indicative of CCR5 co-receptor usage by the virus, and LINC00422 is indicative of CXCR4 co-receptor usage by the virus.

14. The method of any one of the preceding claims, wherein the lncRNA is detected in the sample by hybridization, amplification, ligand-binding, or a functional assay.

15. The method of claim 16, which comprises contacting an aliquot of the biological sample with a nucleic acid probe, a nucleic acid primer, or a ligand, characteristic of said at least one lncRNA, and determining the presence or amount of complexes formed between said probe, primer, or ligand and nucleic acids in the aliquot.

16. The method of claim 17 or 18, wherein the biological sample is treated prior to the determination, preferably by dilution, or concentration, or enrichment for lncRNAs, or for lysing or disrupting cells, or for protecting RNAs.

17. The method of any one of the preceding claims, wherein the biological sample is a biological fluid containing cells, preferably the biological sample is or comprises blood or blood cells.

18. The method of any one of the preceding claims, wherein the subject is a human subject.

19. An agent that modulates a lncRNA as defined in claim 1, for use in the treatment of an infectious disease.


SEQUENCE LISTING

<110>  PRESTIZIA

<120>  Methods for detecting an infectious agent

<130>  B1623EP00

<160>  5

<170>  PatentIn version 3.3

<210>  1
<211>  119
<212>  DNA
<213>  Homo sapiens

<400>  1
gaaacagaca aatatcaaaa aagacaaaga agggcattac ataatggtaa aggtttcagt      60

tcaacaaaga aaaatctcta actattataa atatataagc acccaacaca ggagcaccc      119


<210>  2
<211>  1096
<212>  DNA
<213>  Homo sapiens

<400>  2
acaagtatga agagaatgtc gggagaggaa gtggtggcta tgagtcagca tgagtcatct      60

cgttccaatg agaatgaagg ctgaggtgtg cgcctttttt tttttttcct tcttagtcgt     120

gtgtacatca ttgggaatgg agggaaataa atgactggat ggtcgctgct ttttaagttt     180

caaattgaca ttccagacaa gcggtgcctg agcccgtgcc tgtcttcaga tcttcacagc     240

acagttcctg ggaaggtgga gccaccagcc tctccttgac aagcaaagtg gatcagcaaa     300

ggctgcagtc accagcatct tttccaacct taatgaactg tatcctcaaa agaacactat     360

cagactggta agacaagcat tattgtccca cttcacagat gaagagcttg ggggccctgt     420

ggatgtgtag gagagtcggc cttccttttt ataggccttt tgctgagctc agaggacaga     480

gcagaagaca aagccgaaag cagccctaag cagacactat cccagcccaa ggagctatgc     540

agacagctcc tgcctagagg agaaacaatt ctctccttca gccaggcatg tggcacagag     600

gtgaacagga aatgcttagg gagacagttc acagatatat atgtacttca cagggcacaa     660

tttaatccat aaatcaatca tggatcactg ctaaagaaaa caaaatatgc tgccactaat     720

ttgccaccac cctgtgagca tgattggatg atgtttattt tgattaggag attgctccat     780

ttataaatct tcaatacatc ctgtccccct aaaacggcat ctggatcttt tgagggttaa     840

aaaaaaaaaa agtgaaattg gatgagggat gagagctgga cttctgtgtg tcccggggtc     900

tctggatcca cagcctggtg cctagcatca gttaagccca cgctggggtc gcatcacatc     960

ccgcaaagcc acgtgctctg tgaggccaac aggagccccg ccgggtggac cagctggaat    1020

ctcagagagc gccgactctg aaaatacccg gctctgcaga agcacgctgg gcccaggggg 1080

cttctagact gacagc 1096

<210> 3
<211> 2213
<212> DNA
<213> Homo sapiens

<400> 3
gccccggggc tcaggggcag cccaggactc tccctcgccc ggctcacggc cctgcctggg 60

gggcgcctcc gggagcagat cacgagccct ggggctcagc cctcaggcgc gtctagctcg 120

gcggtcaccc cagtgccggg aggaccctga agacgcgccc caggcggccc tactcagagc 180

ctcctcccaa ggcccaggaa tgcggcgctg tcggaggtgt gcgcggtggc cacaccgctg 240

tccgggtccc caaagcgggc cccgctccca cttctcgcct tggccccgaa ccctgggtcc 300

agccccagcg ctttgtgtgc gaacaccgct ccgccccgga cccagctccg ccttggggcc 360

cctctctgcc tgcccctccg tgcccgacta cactgcttcc cctccggcgg gcgactcagc 420

ccggtccatc gtggcggctt ccagggcggc aggctccggg agtactcccg gggccggctc 480

caaggactgt tcccctcctc cccactccca ctccgcggcg gcggcgggcg agagcggcga 540

catagggcca gggtccggag cggtggaggc tcctggccgg ggagcacgtc gccccacccg 600

gcaacgcgag gatggtggcg gcgcagtcgg ctgctttggg gtctcaaggc acaggggacg 660

cgaggcacag atgtcccaca gcagccactg cggctcccgc agctgctccg ccgccgctgc 720

ccgcccctcc ctgctgcagc tggcgtgatg gcagcggcag ctctggacgc cccactgctg 780

ccaccatcag ccttgtgaaa tagcttgaaa gatctagcca cgtgagcagg acagaagtgc 840

acaacaacca tatcttgatt tctgtggacc aggtggggcc cctgccagcc ttgctagaca 900

gacccaggtg aacagtcctt ctaggggatc tcatcaccag gcaagcacgt ggtacgagaa 960

gagcagtcat taggaaggcc atttggaaaa gcacatcctc tctgttcacg tgagatattt 1020

tacatcctca ttcctcatcg caagcttcct gggatttgga gtgtcacaga caagagggtt 1080

gggggaggcc agtaggtatg gatttgttta tattaaaatg agcatatgaa tatttatatg 1140

tttatattaa aacatatatg tttgtttata ttacaatgag catatgaata tttatatgtt 1200

tatattaaaa catatatgtt tgtttatatt aaaatgagca tatgaatatt tctgtatact 1260

tcagataaac attcttttcc ataaataagc ttcatcatcc agaagccatg ttgaaagttg 1320

gtaatcaagg ataggaagtg tttccaaggg ttgtcagtga ttaaatcaac cttaccttag 1380

catacatgta tgagagaagt tagataaatt atacatagag agatagctag atagataata 1440

gataaataag gtagatgaca gattagatac agatgataaa cagatatgct atcagtctag 1500

atctattact cacagtcagt cgtatcacag tgtaattcca tcaacttcat ttgcagcaaa 1560

agaacagccc ctgatgtacg cctgtttcct gaatatttgg aaatattaat attaatatag 1620

atagagtcag cttgccagta taaagaatct atctctcact ataggcagag ccatgtgttg 1680

gatattatgg aggaatatgt attattttaa tcgtttaaaa gactaataaa cttctaatct 1740

acttttggga gcctctgctc aagttcctga ggttcctact ctaaaatcca catcaccctc 1800

caactgctcc ctatgtgagc cattagggag cacaatgaat tagagaatga atcctattag 1860

cccactaaaa aattatcttt caaaggatga agactgatgc taaggaaaaa gagatgaggc 1920

ctccctggtt ccgcattatt actccttcct ttcttgggtt acggattgaa cactgacctt 1980

ttctttcact cttctgcaag acaaccccat aagaaagctg aaacctgctt ttgtgaaagc 2040

ctatgattac tataagacag gcacagagca agaaaatgtt tgaggcttgt gtctataaac 2100

aagacttggc tggaatcccc agacccacga ctttttaaaa ggatattttg tccctgcaat 2160

acagatactg aatgaggttg gcaaataaat atgtatttct agagactcta taa 2213

<210> 4
<211> 1178
<212> DNA
<213> Homo sapiens

<400> 4
tcatcctgtc ctcacaatgg ctggtgtgga gacccacaga cttcatcctt aggacggacc 60

ttcaggcacc caggctgcct actgacctcc ctggcatggg tgggaaccgg gagctcgttg 120

gtaagcagga ggattcagga ctatcgactg aaccccacct cctctgtggc acgcaccgtg 180

ctgtgtcttt gcagtcatca tctcatcacc tctgcacagg aagattatct cccccatttt 240

gtgatgagga aatggaggct ccaggtggaa agatgcaacc tgggccagag gcccctggag 300

gttgtgcctg aacccagaca gatcacacaa acctgagcag ccattagccg ctgtgtgggc 360

tcctttgcac tgtggggctg ctcctgggga tatttacgtt gtgaactggt aaaatctgaa 420

ctgcagaata tcaatttcca gacattcttc ctagatgatg ccgtctgcca cggagaccat 480

gtagatggat tgaatgggga tatgatgagg atgaccaccc ctgcatttgc taagttcttg 540

ggagtggcat cgatctcagc cattccactg gaatagggtc aggaaacaaa acgtgagggc 600

tctaccagct gctcagtcta tcctttccaa ttgagcacac tggaactgag gaagcaaaga 660

cagggtgagc cctaacaccc tcatcatcac tgctgagcct gccttgacaa gaactccatt 720

tatcacccgc ctccataata gcccattcta actagctgtc atcgtggcat ttcaggttct 780

gctgatgtca ttataatgga ccttaactag tgggctgaag gcaatgaggg gatgtgagga 840

tgcatcttgt gaaggacaaa agtcatcctg taaggattca tgagacctga gcagccagcc 900

agccctgaaa tggttggaac aggagaaaga agaagagctc gacagatacc ctgcctgggc 960

tggcagctag ctgcaccaca agctggccag ctgacagcgg gcacatccct tcatcaccca 1020

gtgcctttct tccctcttgt gaaaatggag atatgctgag aaggttactt cagctcatcc 1080

agagaaagct tcttcagcta actaggctgt agaaagggcc tacagcctaa attttcaaaa 1140

acaagttaat gccagaatat aaactccgtg aagtaaca 1178

<210> 5
<211> 1096
<212> DNA
<213> Homo sapiens

<400> 5
acaagtatga agagaatgtc gggagaggaa gtggtggcta tgagtcagca tgagtcatct 60

cgttccaatg agaatgaagg ctgaggtgtg cgcctttttt tttttttcct tcttagtcgt 120

gtgtacatca ttgggaatgg agggaaataa atgactggat ggtcgctgct ttttaagttt 180

caaattgaca ttccagacaa gcggtgcctg agcccgtgcc tgtcttcaga tcttcacagc 240

acagttcctg ggaaggtgga gccaccagcc tctccttgac aagcaaagtg gatcagcaaa 300

ggctgcagtc accagcatct tttccaacct taatgaactg tatcctcaaa agaacactat 360

cagactggta agacaagcat tattgtccca cttcacagat gaagagcttg ggggccctgt 420

ggatgtgtag gagagtcggc cttccttttt ataggccttt tgctgagctc agaggacaga 480

gcagaagaca aagccgaaag cagccctaag cagacactat cccagcccaa ggagctatgc 540

agacagctcc tgcctagagg agaaacaatt ctctccttca gccaggcatg tggcacagag 600

gtgaacagga aatgcttagg gagacagttc acagatatat atgtacttca cagggcacaa 660

tttaatccat aaatcaatca tggatcactg ctaaagaaaa caaaatatgc tgccactaat 720

ttgccaccac cctgtgagca tgattggatg atgtttattt tgattaggag attgctccat 780

ttataaatct tcaatacatc ctgtccccct aaaacggcat ctggatcttt tgagggttaa 840

aaaaaaaaaa agtgaaattg gatgagggat gagagctgga cttctgtgtg tcccggggtc 900

tctggatcca cagcctggtg cctagcatca gttaagccca cgctggggtc gcatcacatc 960

ccgcaaagcc acgtgctctg tgaggccaac aggagccccg ccgggtggac cagctggaat 1020

ctcagagagc gccgactctg aaaatacccg gctctgcaga agcacgctgg gcccaggggg 1080

cttctagact gacagc 1096